Europäisches Patentamt

European Patent Office

Office européen des brevets

(19)

(11) **EP 0 723 009 A1**

(12) **DEMANDE DE BREVET EUROPEEN**

(43) Date de publication:
24.07.1996 Bulletin 1996/30

(51) Int Cl.6: **C12M 3/08**

(21) Numéro de dépôt: 96420009.1

(22) Date de dépôt: 09.01.1996

(84) Etats contractants désignés:
AT BE DE ES FR GB IT NL SE

(30) Priorité: 17.01.1995 FR 9500677

(71) Demandeur: HOSPAL R&D Int.
69881 Meyzieu Cedex (FR)

(72) Inventeur: Prevost, Philippe
F-69004 Lyon (FR)

(74) Mandataire: Lejeune, Daniel
Hospal Service Brevets,
B.P. 21
F-69881 Meyzieu Cédex (FR)

(54) **Dispositif de digestion d'organe et procédé pour isoler des cellules ou amas de cellules d'interêt biologique à partir d'un organe**

(57) Un dispositif de digestion d'un organe en vue d'en extraire des cellules ou amas de cellules d'intérêt biologique comprend :

- une enceinte (1,2)

- un conteneur (5) disposé dans l'enceinte (1,2) pour contenir l'organe, délimitant une première chambre située à l'extérieur du conteneur et une deuxième chambre à l'intérieur du conteneur (5) ;

- des moyens (6) pour faire tourner le conteneur (5) autour d'un axe, et

- des moyens de communication sélectifs (23) entre la première chambre et la deuxième chambre pour permettre le passage de liquide de la première chambre vers la deuxième chambre et le passage de fragments d'organe de la deuxième chambre vers la première chambre.

Fig. 1

**Description**

La présente invention a pour objet un dispositif de digestion d'organe et un procédé pour isoler des cellules ou amas de cellules d'intérêt biologique à partir d'un organe.

On sait pallier aujourd'hui la déficience totale ou partielle, permanente ou temporaire, de certains organes par des dispositifs capables de remplir au moins les fonctions vitales de ces organes, telles que des fonctions de pompage, d'échange gazeux, d'épuration par filtration et par dialyse, qui sont assurées naturellement par le coeur, les poumons et les reins.

On sait aussi pallier la déficience de certains organes dont au moins l'une des fonctions vitales consiste à sécréter une substance indispensable au métabolisme, par injection à ces patients d'un succédané préalablement synthétisé ou isolé d'une source animale. C'est le cas par exemple de l'insuline, qui est naturellement sécrétée par des amas de cellules logées dans le pancréas.

Toutes les techniques qui viennent d'être évoquées ne sont cependant que des palliatifs qui, s'ils permettent la survie des patients, sont impropres à les prémunir contre certains troubles, parfois incapacitants, tels que l'anémie des insuffisants rénaux chroniques, ou la cécité et l'athérosclérose des diabétiques.

Pour certains organes, tels les reins, une façon de remédier à l'efficacité limitée des palliatifs artificiels, consiste à recourir à la greffe d'organe. La greffe d'organe est aussi la seule possibilité de sauver les patients qui ont perdu l'usage d'un organe, tel que le foie, dont on ne sait pas actuellement reproduire les fonctions vitales par des moyens artificiels. La greffe d'organe reste cependant une technique d'application limitée, et à cause du nombre restreint de donneurs, et à cause des réactions immunitaires qui rendent en particulier impossible la transplantation d'organe d'une espèce animale à une autre.

Une troisième voie pour pallier la déficience de certains organes, qui fait actuellement l'objet de recherches actives, pourrait résider dans la suppléance naturelle des fonctions de ces organes au moyen de dispositifs conçus pour contenir un matériel biologique actif. La fabrication de ces organes bioartificiels, telle qu'on l'imagine aujourd'hui, comprend deux étapes principales : la première de ces étapes consiste à isoler, à partir d'un organe, qui pourrait, de préférence, être celui d'un animal, des cellules (hépatocytes, par exemple) ou amas de cellules (îlots de Langerhans, par exemple) remplissant des fonctions qui ne sont plus assurées chez les patients ayant perdu l'usage de cet organe. La deuxième étape de la fabrication d'un organe bioartificiel consiste à encapsuler les cellules ou amas de cellules dans une enveloppe faite d'un matériau biocompatible semi perméable destiné à isoler les cellules de l'organisme receveur pour prévenir les réactions immunitaires, tout en permettant les échanges nécessaires à la survie des cellules et à la sécrétion des substances dont l'organisme receveur a besoin.

L'invention a trait à la première étape de cette fabrication, dont on comprend qu'elle est cruciale puisque de l'aptitude à extraire efficacement d'un organe les cellules d'intérêt biologique tout en préservant leurs facultés de sécrétion dépend le développement des organes bioartificiels.

Le brevet US 5 079 160 décrit un dispositif et un procédé d'isolement de cellules d'intérêt biologique à partir d'un organe, en particulier d'îlots de Langerhans à partir d'un pancréas.

Le dispositif comprend une chambre de digestion formée par un conteneur divisé en un compartiment inférieur et un compartiment supérieur par un treillis dont les mailles sont choisies pour permettre le passage des cellules d'intérêt biologique et pour retenir des fragments d'organe de taille supérieure. Le compartiment supérieur est muni d'un orifice de sortie. La compartiment inférieur est muni d'orifices d'entrée pour un liquide et il contient des billes destinées à hâter la désagrégation d'un organe placé dans ce compartiment et à agiter le liquide dans lequel baigne l'organe. Le conteneur est prévu pour être monté dans un dispositif agitateur ayant pour effet de remuer les billes dans le compartiment inférieur et de secouer un organe placé dans ce compartiment.

La chambre de digestion est reliée à un circuit de circulation de liquide en boucle fermée et un circuit de circulation de liquide en boucle ouverte. Les deux circuits ont une partie commune comprenant une pompe de circulation, un dispositif de refroidissement disposé en aval de la chambre et un dispositif de chauffage disposé en amont de la chambre.

Le procédé d'isolement d'îlots de Langerhans décrit dans ce document consiste à injecter dans l'organe un liquide de digestion propre à attaquer le tissu conjonctif et à provoquer la désagrégation de l'organe nécessaire à la libération des cellules d'intérêt biologique. L'organe distendu par le liquide de digestion est alors placé dans le compartiment inférieur de la chambre de digestion, puis la chambre est remplie de liquide de digestion. Du liquide de digestion est recirculé de façon continue dans la chambre de digestion au moyen du circuit en boucle fermé, tandis que celle-ci est secouée pour y remuer l'organe et y agiter le liquide, effets amplifiés par les billes. Le liquide de digestion est refroidi aussitôt sorti de la chambre pour en neutraliser les effets sur des cellules éventuellement libérées et il est réchauffé juste avant son retour dans la chambre pour y avoir son efficacité maximale. Des prélèvements sont effectués régulièrement dans le liquide sortant de la chambre. Dès que des cellules d'intérêt biologique sont observées dans un échantillon, un liquide de rinçage est mis en circulation dans la chambre au moyen du circuit de circulation en boucle ouverte et les cellules, amas de cellules et fragments d'organe pouvant passer au travers du treillis sont recueillis. Les cellules sont ensuite isolées par centrifugation sur gradient continu.

Le dispositif et le procédé qui viennent d'être évoqués présentent plusieurs inconvénients. En premier lieu, la mise en oeuvre du procédé soumet à la fois l'organe à des contraintes mécaniques dues à l'action des billes et à des contraintes de pression, dues à la circulation de liquide dans la chambre qui provoque, dès que l'organe commence à se désagréger, un colmatage partiel du treillis. L'expérience a montré que au moins certaines cellules, les îlots de Langerhans en particulier, étaient très sensibles à ce type de contraintes. Par ailleurs, les techniques décrites dans ce brevet sont des techniques de laboratoires qui paraissent difficilement adaptables à une exploitation industrielle. En particulier l'étape de rinçage, en circuit ouvert, de la chambre de digestion pour en déloger les cellules, amas de cellules et fragments de tissu conjonctif de taille inférieure aux mailles du treillis, nécessite un volume relativement important de liquide de rinçage. Or le liquide recueilli doit être ensuite centrifugé pour purifier les cellules et la contenance des dispositifs de séparation de cellules disponibles sur le marché est réduite, de sorte que l'étape de purification est longue, beaucoup plus qu'il ne serait acceptable dans un processus industriel ou semi industriel.

Un but de la présente invention est de proposer une technique d'obtention de cellules d'intérêt biologique à partir d'un organe qui soumette les cellules à un minimum de contraintes physiques et qui soit adaptable à une exploitation industrielle.

Pour atteindre ce but, on prévoit, conformément à l'invention, un dispositif de digestion d'au moins un organe ou d'une partie d'organe en vue d'en extraire des cellules ou amas de cellules d'intérêt biologique comprenant:

- une enceinte, et
- des moyens d'agitation mécanique pour favoriser la désagrégation d'un organe soumis à l'action d'un milieu de digestion biologique,

caractérisé en ce que les moyens d'agitation mécanique comportent des moyens pour entraîner en rotation au moins partielle sur lui-même un organe placé dans l'enceinte.

Selon un premier mode de réalisation de l'invention, les moyens pour entraîner en rotation un organe placé dans l'enceinte comprennent un moteur pouvant être couplé à l'enceinte pour faire tourner l'enceinte autour d'un axe sensiblement horizontal.

Selon un second mode de réalisation de l'invention, les moyens pour entraîner en rotation un organe placé dans l'enceinte comprennent :

- un conteneur disposé dans l'enceinte pour contenir l'organe, délimitant une première chambre située à l'extérieur du conteneur et une deuxième chambre à l'intérieur du conteneur ;
- des moyens pour faire tourner le conteneur autour d'un axe, et
- des moyens de communication sélectifs entre la première chambre et la deuxième chambre pour permettre le passage de liquide de la première chambre vers la deuxième chambre et le passage de fragments d'organe de la deuxième chambre vers la première chambre.

Grâce à ce dispositif, l'organe n'est pas heurté contre les parois de l'enceinte ni ne reçoit d'impacts d'aucune sorte et comme il est cependant remué de façon sensible, sa désagrégation est facilitée. Par ailleurs, le mouvement de l'organe dans l'enceinte brasse le liquide de digestion suffisamment pour homogénéiser ce dernier, de sorte qu'avec le dispositif selon l'invention, il n'est pas indispensable de faire circuler le liquide dans une boucle fermée sur l'enceinte pendant la digestion. On peut éviter ainsi de soumettre les cellules ou amas de cellules aux contraintes de pression indésirables causées notamment par une pompe de circulation.

Selon une caractéristique de l'invention, le conteneur a sensiblement la forme d'un tambour ayant deux parois d'extrémité sensiblement circulaires jointes par une paroi latérale tubulaire ; le tambour est entraîné en rotation autour de son axe de symétrie, qui est disposé sensiblement à l'horizontale.

Selon une autre caractéristique de l'invention, les moyens de communication sélectifs comprennent au moins une portion d'une paroi du conteneur constituée par un treillis. Lorsque le conteneur est un tambour, sa paroi tubulaire est avantageusement constituée par un treillis.

Selon encore une autre caractéristique de l'invention, le dispositif selon l'invention comporte des moyens pour régler la température d'un liquide dans l'enceinte comprenant :

- une chemise entourant au moins partiellement l'enceinte,
- des moyens pour faire circuler un liquide dans la chemise, et
- des moyens pour régler la température du liquide mis en circulation dans la chemise.

Selon l'invention, on prévoit aussi un procédé d'isolement de cellules ou d'amas de cellules d'intérêt biologique à partir d'un organe ou d'une partie d'organe comprenant les étapes de :

- injecter dans l'organe un liquide de digestion apte à provoquer une désagrégation du tissu conjonctif enserrant les cellules d'intérêt biologique ;
- placer l'organe dans une enceinte et remplir l'enceinte de liquide ;
- agiter l'organe dans l'enceinte pendant la digestion
- vidanger le contenu de l'enceinte lorsque la digestion est achevée
- séparer les cellules d'intérêt biologique du contenu de l'enceinte

caractérisé en ce que l'étape d'agitation de l'organe consiste à soumettre l'organe à un mouvement de rotation au moins partielle sur lui-même jusqu'à sa désagrégation.

Selon une caractéristique de l'invention, le temps s'écoulant entre le début de l'étape d'injection de liquide de digestion dans l'organe et le début de l'étape de vidange du contenu de l'enceinte a une durée fixe prédéterminée.

On a en effet observé que, pour un organe donné et un type de liquide de digestion donné, la désagrégation de l'organe se produit pendant un intervalle de temps bref, après une durée de digestion sensiblement constante qu'il est possible de déterminer empiriquement. Conformément à l'invention, il n'est donc pas nécessaire d'effectuer des prélèvements réguliers dans la chambre pour déterminer quand les premières cellules ou les premiers amas de cellules d'intérêt biologique sont libérés du tissu conjonctif.

Selon une autre caractéristique de l'invention, l'enceinte est vidangée par gravité. Grâce à cette disposition, on peut éviter de soumettre les cellules ou amas de cellules aux contraintes de pression indésirables causées par une pompe.

D'autres caractéristiques et avantages de l'invention apparaîtront à la lecture de la description qui va suivre. On se reportera aux dessins annexés sur lesquels :

La figure 1 est une vue en coupe selon un plan vertical d'un premier mode de réalisation d'un dispositif de digestion selon l'invention ;

La figure 2 est une vue en coupe schématique, selon un plan vertical, d'un deuxième mode de réalisation d'un dispositif de digestion selon l'invention ;

La figure 3 est une vue en coupe schématique, selon un plan vertical, d'un troisième mode de réalisation d'un dispositif de digestion selon l'invention.

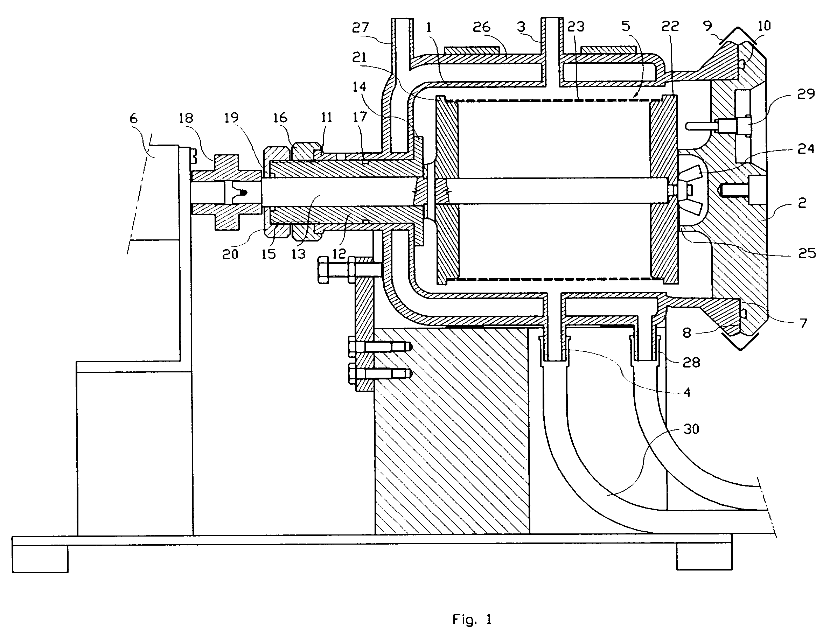

Le dispositif de digestion représenté sur la figure 1 comprend une enceinte constituée par un récipient 1 pouvant être fermé de façon étanche par un bouchon 2. L'enceinte est munie de deux voies de communication avec l'extérieur permettant l'introduction et l'évacuation d'un liquide. L'une de ces voies est formée par une tubulure 3 connectée à une partie haute du récipient 1 et l'autre voie est formée par une tubulure 4 connectée à une partie basse du récipient 1. Le récipient 1 a la forme d'une cuve cylindrique dont l'ouverture, sur laquelle s'emboîte le bouchon 2, est située dans un plan sensiblement vertical. Le bord du récipient 1 présente une surface radiale périphérique 7 contre laquelle vient s'appliquer une surface radiale périphérique correspondante 8 du bouchon 2, lorsque le bouchon 2 est maintenu enfoncé sur le récipient 1 par un collier 9 à mécanisme d'attache rapide (non représenté). Un joint disposé dans une gorge circulaire 10 pratiquée dans le bouchon 2 assure l'étanchéité de l'enceinte lorsque le collier 9 est en place.

Conformément à l'invention, le dispositif de digestion comprend en outre des moyens pour entraîner en rotation partielle sur lui-même un organe placé dans l'enceinte. Dans le mode de réalisation de la figure 1, ces moyens sont constitués par un conteneur 5 monté dans l'enceinte de façon à pouvoir être entraîné en rotation par un moteur 6 disposé à l'extérieur de l'enceinte.

A cet effet, le fond du récipient 1 comprend une ouverture circulaire centrée sur l'axe longitudinal du récipient, à partir de la périphérie de laquelle s'étend, vers l'extérieur, un fourreau tubulaire 11 servant au montage, sur le récipient 1, d'un palier 12 pour un arbre 13 d'entraînement du conteneur 5. Le palier 12 est constitué par un manchon tubulaire muni, à une extrémité, d'un collet 14 et, à son autre extrémité, d'un filet 15. Le diamètre extérieur du palier 12 est légèrement inférieur au diamètre intérieur du fourreau 11. Le palier 12 est engagé dans le fourreau 11 par l'intérieur du récipient 1 jusqu'à ce que le collet 14 vienne en butée contre le fond du récipient. Un écrou 16 est vissé sur l'extrémité filetée du palier 12 jusqu'à buter contre l'extrémité libre du fourreau 11 de façon à immobiliser le palier 12 par rapport au récipient 1. L'étanchéité entre le fourreau 11 et le palier 12 est assurée par un joint disposé dans une gorge circulaire 17 ménagée à la périphérie du palier 12.

Le palier 12 sert de support à l'arbre 13 dont une extrémité est connectable, par l'intermédiaire d'un organe d'accouplement 18, au moteur 6. L'étanchéité entre le palier 12 et l'arbre 13 est assurée par un joint disposé dans un lamage 19 pratiqué à l'extrémité du palier 12. Le joint est maintenu en place par une virole 20 vissée à l'extrémité du palier 12.

L'arbre 13 s'étend à l'intérieur de l'enceinte jusqu'à proximité du bouchon 2. Outre sa fonction de transmission du mouvement rotatif engendré par le moteur 6, il sert à l'assemblage du conteneur 5, lequel a la forme d'un tambour constitué par deux parois d'extrémité 21, 22 sensiblement circulaires jointes par une paroi latérale tubulaire 23. Une première paroi circulaire 21 est goupillée à l'arbre 13 de façon que, lorsque l'arbre est en place dans l'enceinte, cette paroi se trouve à proximité du fond du récipient 1. L'autre paroi circulaire 22 forme un bouchon amovible pouvant s'emboîter dans la paroi latérale 23. Elle présente un trou en son centre pour le passage de l'extrémité filetée de l'arbre 13, de sorte que le conteneur 5 peut être fermé par vissage d'un écrou papillon 24 sur l'extrémité de l'arbre.

Afin de pouvoir sortir le conteneur 5 du récipient 1, l'arbre 13 n'est pas assujetti axialement au moteur 6. Pour empêcher, en fonctionnement, le déplacement axial du conteneur 5, le bouchon 2 est muni, sur sa face interne, d'une cale tubulaire 25 contre laquelle vient buter la paroi circulaire 22 amovible du conteneur 5.

Le conteneur 5 délimite deux chambres dans l'enceinte, une première chambre située à l'extérieur du conteneur et une deuxième chambre située à l'intérieur du conteneur, destinée à recevoir un ou plusieurs organes ou une partie d'organe à digérer. Des moyens de communication sélectifs permettent le passage de liquide de la première chambre vers la deuxième chambre et de liquide et de fragments d'organe de la seconde chambre vers la première chambre. Dans ce dispositif de digestion représenté sur la figure 1, ces moyens de communication sélectifs comprennent un treillis dont est formée la paroi tubulaire 23 du conteneur 5.

Le dispositif de digestion selon l'invention comprend en outre des moyens pour régler la température du contenu de l'enceinte. Ces moyens de réglage de température comprennent une chemise 26 entourant l'enceinte, munie d'une tubulure d'entrée 27 et d'une tubulure de sortie 28 pour permettre la circulation, dans la chemise 26, d'un liquide dont la température est ajustée par des moyens de chauffage ou de refroidissement (non représentés) en fonction d'une température de consigne et des informations fournies par une sonde de température 29 disposée dans l'enceinte. Dans le mode de réalisation représenté, la sonde de température 29 est montée dans le bouchon 2 de l'enceinte.

La mise en oeuvre de ce dispositif de digestion est la suivante. Le conteneur 5 étant sorti du récipient 1, on y place les organes ou parties d'organe à digérer, qui ont été préalablement distendus au moyen d'une solution de digestion constituée par une enzyme propre à attaquer le tissu conjonctif de l'organe, dissoute dans une solution saline (solution de Hanks, par exemple). Après avoir fermé le conteneur 5, on enfile l'arbre 13 dans le palier 12 de façon à réaliser le couplage de l'arbre 13 à l'arbre de sortie du moteur 6. On ferme ensuite l'enceinte en emboîtant le bouchon 2 dans le récipient 1, et l'on solidarise récipient 1 et bouchon 2 au moyen du collier 9. La tubulure d'accès inférieure 4 de l'enceinte étant fermée (par exemple par un clamp placé sur la canalisation de vidange 30) on remplit l'enceinte, par la tubulure d'accès supérieure 3, avec une solution saline préalablement portée à température appropriée. Le moteur 6 est alors mis en marche pour faire tourner le conteneur 5, tandis qu'un liquide de régulation de température est mis en circulation dans la chemise 26 pour maintenir la température dans l'enceinte sensiblement égale à une température de consigne. Lorsque la digestion est considérée achevée, le moteur 6 est stoppé. A ce stade, le conteneur 5 contient principalement des fragments d'organe dont la taille est inférieure à celle des mailles du treillis formant la paroi tubulaire 23 du conteneur. Le contenu de l'enceinte est vidangé et les cellules ou amas de cellules libérés par la digestion sont isolées par un procédé qui sera décrit plus loin. Selon le type d'organe à digérer, la composition du liquide de digestion utilisé et la place occupée par le conteneur 5 dans l'enceinte 1, 2, le liquide présent dans l'enceinte peut être recirculé continûment pour, le cas échéant, en améliorer l'homogénéité Dans ce cas, les tubulures d'accès 3, 4 sont reliées l'une à l'autre par une canalisation sur laquelle est disposée une pompe.

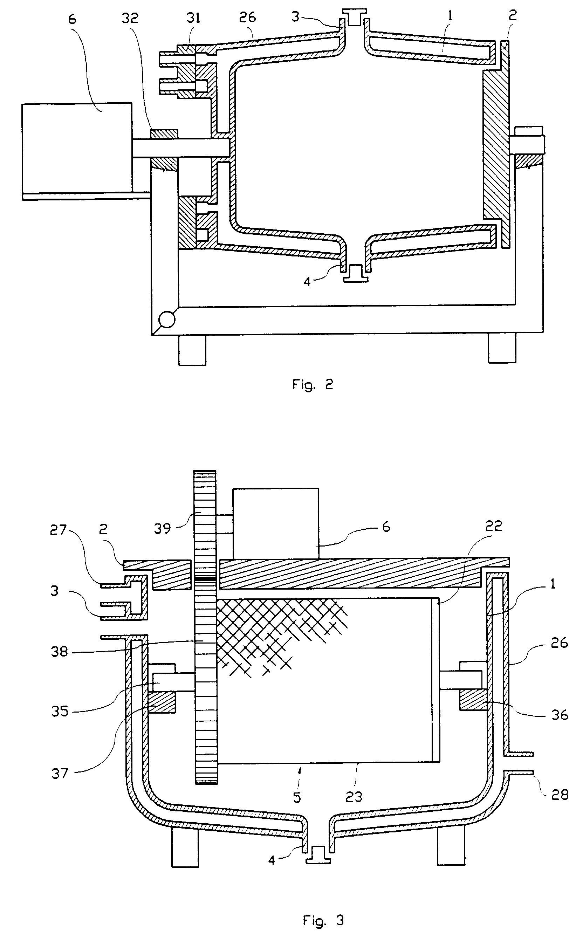

Sur les figures 2 et 3, qui représentent deux autres modes de réalisation de l'invention, les mêmes chiffres de référence ont été repris pour indiquer des organes identiques ou similaires.

La figure 2 représente un autre mode de réalisation du dispositif de digestion selon l'invention qui se différencie de celui de la figure 1 essentiellement en ce qu'il ne comprend pas de conteneur rotatif disposé à l'intérieur d'une enceinte fixe. Ici, c'est l'enceinte elle-même qui est entraînée en rotation autour d'un axe sensiblement horizontal. Un distributeur rotatif 31 permet de faire circuler dans la chemise 26 le liquide de régulation de température de l'enceinte. L'enceinte est montée sur un support articulé 32 permettant de faire pivoter l'enceinte d'une première position où l'ouverture du récipient 1 est dans un plan horizontal et une deuxième position où l'ouverture du récipient 1 est dans un plan vertical (position représentée sur la figure). La première position est prévue pour le remplissage du récipient 1 avec les organes à digérer et la solution saline. La deuxième position est la position de fonctionnement du dispositif de digestion. Le remplissage de l'enceinte avec la solution saline peut être aussi effectué lorsque l'enceinte est dans cette seconde position. Une fois la digestion achevée, la vidange de l'enceinte se fait avantageusement dans cette deuxième position, par gravité : la tubulure d'accès se trouvant alors à la partie haute de l'enceinte est ouverte pour permettre l'entrée d'air dans l'enceinte et la tubulure d'accès disposée à la partie basse de l'enceinte étant reliée à un réservoir de recueil.

La figure 3 représente un autre mode de réalisation du dispositif de digestion selon l'invention qui se différencie de celui de la figure 1 essentiellement en ce que l'ouverture du récipient 1 est disposée dans un plan horizontal. Le conteneur 5 comprend un axe longitudinal 35 dont les deux extrémités reposent sur des paliers 36, 37 ayant la forme d'un U ouvert vers le haut, fixés à l'intérieur du récipient 1. A une de ses extémités, le conteneur 5 porte une couronne dentée 38 destinée à coopérer avec une roue dentée 39 entrainée par un moteur 6 fixé sur le couvercle 2 du récipient 1. Un intérêt de ce dispositif est qu'il permet de porter l'enceinte remplie de solution saline à la température requise avant d'y introduire les organes à digérer.

Les trois modes de réalisation du dispositif selon l'invention qui viennent d'être décrits permettent de mettre en oeuvre le procédé d'isolement de cellules suivant :

L'organe dont on veut extraire des cellules ou amas de cellules d'intérêt biologique (îlots de Langerhans pour le pancréas, par exemple), est prélevé sur l'organisme donneur dans des conditions de stérilité requises puis transféré sur le lieu de traitement dans un liquide de conservation à une température légèrement supérieure à 0°C, environ 4°C par exemple. L'organe est alors apprêté pour l'étape de digestion qui va suivre, c'est-à-dire est débarrassé de la graisse et des tissus étrangers qui l'entourent éventuellement.

L'extraction des cellules d'intérêt biologique enserrées dans le tissu conjonctif est provoquée par une digestion enzymatique associée à une agitation mécanique. On commence par injecter dans l'organe, par un conduit naturel de celui-ci de préférence, un volume déterminé de liquide de digestion (liquide constitué par une solution saline dans laquelle est dissoute une enzyme capable de dépolymériser les protéines du tissu conjonctif, typiquement la collagénase) à une température favorisant une digestion optimale (environ 37°C). L'organe est placé dans l'enceinte du dispositif de digestion qui, soit contient déjà, soit est remplie alors, de solution saline à la même température. Cette solution saline peut être la même que celle qui est utilisée pour préparer le liquide de digestion. Pendant toute la durée de la digestion, la température du contenu de l'enceinte est maintenue à ce niveau optimal au moyen du liquide mis en circulation dans la chemise 26 de l'enceinte.

Dès que l'organe baigne dans la solution saline et jusqu'à la fin de l'étape de digestion, l'organe est agité de façon à favoriser sa désagrégation. Conformément à l'invention, l'agitation à laquelle l'organe est soumis ne s'accompagne ni de heurts, ni d'impacts de corps étrangers d'aucune sorte, en ce qu'elle consiste en un mouvement de rotation, au moins partielle, de l'organe sur lui-même engendré par la rotation du conteneur (figures 1, 3) ou de l'enceinte (figure 2). Ce mouvement de rotation peut être continu, toujours dans le même sens, ou alterné dans un sens puis dans l'autre sens pendant au moins un tour dans chaque sens, ou un mouvement de rotation partielle alterné, un demi-tour dans un sens puis un demi-tour dans l'autre sens par exemple.

Conformément à l'invention, la durée pendant laquelle l'organe est digéré est avantageusement choisie fixe, depuis le moment où il est distendu avec le liquide de digestion jusqu'au moment où la chambre de digestion est vidangée. On part de l'observation que la désagrégation de l'organe soumis au liquide de digestion n'est pas un phénomène progressif, mais qu'elle intervient sur un espace de temps assez bref. Il suffit donc, pour un organe et un type de digestion (nature du liquide, forme de l'agitation) donnés, de déterminer un temps de digestion optimal au bout duquel le tissu conjonctif s'est désagrégé suffisamment pour libérer les cellules ou groupes de cellules d'intérêt biologique, sans que le liquide de digestion n'ait eu le temps de les attaquer.

Grâce à la conformation du dispositif de digestion décrit plus haut, il n'est pas nécessaire, pour homogénéiser le liquide contenu dans l'enceinte, de faire circuler celui-ci au moyen d'une pompe disposée sur une canalisation bouclée sur l'enceinte. Dans les modes de réalisation des figures 1 et 3, l'homogénéisation du liquide dans l'enceinte résulte à la fois de la rotation du conteneur et du mouvement de l'organe à l'intérieur de celui-ci. On peut aussi adjoindre au conteneur 5 des ailettes s'étendant dans la première chambre. Dans le mode de réalisation de la figure 2, le brassage du liquide peut être obtenu en choisissant la vitesse de rotation de l'enceinte de façon que l'organe reste plaqué contre la paroi tubulaire de celle-ci jusqu'à mi-hauteur de l'enceinte, par exemple, pour s'en détacher alors sous l'effet de son propre poids et retomber dans la partie basse de l'enceinte en provoquant des remous dans le liquide de digestion.

A la fin du temps de digestion, le contenu de l'enceinte est vidangé, de préférence par gravité, dans un récipient, puis l'enceinte est rincée avec une solution saline, qui est également recueillie dans ce récipient.

Le contenu du récipient de recueil qui est constitué d'un mélange de liquide de digestion et de liquide de rinçage, de fragments de tissu conjonctif et de cellules ou d'amas de cellules d'intérêt biologique est alors passé sur un tamis calibré pour laisser passer les cellules ou amas de cellules et retenir une proportion variable de fragments de tissu conjonctif, selon l'organe considéré.

Puis le filtrat est centrifugé pour séparer l'organe digéré du liquide de digestion et du liquide de rinçage. Le culot de centrifugation est mis en suspension dans un liquide de conservation.

Enfin les îlots sont purifiés par centrifugation sur un gradient de densité continu. Le principe de la séparation de cellules sur gradient de densité continu est expliqué notamment dans le brevet américain 5 368 542.

Exemple :

On a réalisé à quatre reprises l'isolement d'îlots de Langerhans à partir d'une portion du lobe splénique de quatre pancréas de porcs (P1, P2, P3, P4). Lors de chaque expérience, 400 ml pour P1, P2, P3, 200 ml pour P4 d'une solution de collagénase à 37°C ont été injectés dans le pancréas par le canal de Wirsung, puis la portion de lobe splénique du pancréas a été placée dans l'enceinte du dispositif de digestion représenté sur la figure 1, à l'intérieur du conteneur rotatif 5. Pour P1, P2, P3, la solution de collagénase contenait 500 mg de collagénase dissous dans 400 ml de de solution de Hanks et Pour P4, la solution de collagénase contenait 500 mg de collagénase dissous dans 200 ml de de solution de Hanks (solution de Hanks, disponible notamment chez la société SIGMA sous la référence H 1387).

Le dispositif utilisé avait les caractéristiques suivantes : l'enceinte, de verre, avait un volume intérieur de 1 l et le conteneur, dont la paroi tubulaire était constituée d'un treillis métallique ayant des mailles carrées de 3 mm de côté, avait un volume intérieur de 0,34 l.

L'enceinte a été complètement remplie de solution de Hanks à 37°C, puis les deux tubulures d'accès 3, 4 à l'enceinte ont été reliées par une canalisation pour former un circuit fermé dans lequel la solution a été mise en circulation à 300 ml/mn (P1), 100 ml/mn (P2), 160 ml/mn (P3, P4). La température dans l'enceinte a été maintenue aux alentours de 37°C, après avoir été atteinte en une dizaine de minutes (à l'exception de P1 et P4). Le temps de digestion total a été fixé à 50 minutes, pendant un peu plus des deux tiers desquels le fragment d'organe a été agité dans l'enceinte, la vitesse de rotation du conteneur ayant été fixée à 40 tours/mn.

A la fin du temps de digestion, le contenu de l'enceinte a été vidangé par gravité dans un récipient de recueil, puis l'enceinte a été rincée avec 500 ml de solution de Hanks. La quasi totalité de la portion du lobe splénique introduite dans la chambre a été digérée (96% en moyenne).

Tableau 1

| N° pancréas | Poids du pancréas (g) | Poids de pancréas à digirer (g) | Poids de pancréas digéré (g) | Poids de pancréas digéré % | Durée de l'agitation (min) |
|---|---|---|---|---|---|
| P1 | 261 | 110 | 102 | 99 | 33 |
| P2 | 255 | 91 | 82 | 90 | 32 |
| P3 | 267 | 90 | 90 | 100 | 34 |
| P4 | 322 | 127 | 119 | 94 | 38 |

Le contenu du récipient de recueil (environ 900 ml), formé de tissu pancréatique digéré en suspension, a ensuite été passé successivement sur deux tamis : un tamis à mailles de 1,25 mm, puis un tamis à mailles de 0,83 mm pour P1 et P2, après une première centrifugation de 5 min à 120 g ; un tamis à mailles de 1 mm puis un tamis à mailles de 0,6 mm pour P3 et P4, avant centrifugation.

Le culot de la première centrifugation a été, pour P1 et P2, après filtration, centrifugé à deux autres reprises, à 120 g, pendant 5 minutes à chaque fois.

Le filtrat pour P3 et P4 n'a été centrifugé qu'une fois, pendant 5 minutes à 120 g pour P3, pendant 2 minutes à 100 g pour P4.

Le culot de centrifugation final (environ 18 ml) a été resuspendu dans environ 145 ml de solution UW modifiée (University of Wisconsin solution, telle que décrite dans Transplantation, Vol. 57, 346-354, N°3, February 1994 ; la solution UW est modifiée en ce qu'elle est dépourvue de hydroxy amidon) et les îlots de Langerhans ont été purifiés sur gradient de densité continu Nycodenz (produit commercialisé par la société Nycomed Pharma) dans une machine de séparation de cellules 2911 COBE, tournant à 2000 tours/mn pendant 8 mn.

Les tableaux 2 et 3 ci-dessous indiquent le résultat des purifications réalisées.

Tableau 2

| N° de Pancréas | Nombre d'I.E (1) déposés sur le gradient | Index d'isolement après digestion | Zone de densité de migration des îlots | Zone de densité de récupération des îlots purifiés |
|---|---|---|---|---|
| P1 | 552 800 | 1,28 | 1,03 - 1,058 | 1,03 |
| P2 | 238 600 | 1,83 | 1,03; 1,056 - 1,098 | 1,03 |
| P3 | 334 000 | 1,51 | 1,03; 1,065 - 1,098 | 1,03 |
| P4 | 734 100 | 2,24 | 1,03; 1,058 - 1,098 | 1,03 |
| Moyenne | 464 900 | 1,72 | Intervalle le plus grand : 1,03 - 1,098 | Intervalle le plus grand : 1,03 |
| Ecart-type | 222 500 | 0,42 | | |

(1) I.E = îlot-équivalent, correspondant à un îlot théorique de 150 $\mu$m de diamètre (nombres arrondis à la centaine).

Tableau 3

| N° de Pancréas | Nombre d'I.E (1) récupérés après purification | Index d'isolement après purif. (2) | Rendement de purif. % (3) | Rendement final par g pancréas digéré (4) | Taux de pureté (%) (5) |
|---|---|---|---|---|---|
| P1 | 425 100 | 1,15 | 77 | 4 170 | 90 |
| P2 | 119 000 | 1,19 | 50 | 1 450 | 74 |
| P3 | 291 800 | 1,04 | 87 | 3 240 | 81 |
| P4 | 703 100 | 3,68 | 96 | 5 530 | 90 |
| Moyenne | 384 800 | 1,77 | 77 | 3 600 | 84 |
| Ecart-type | 246 500 | 1,28 | 20 | 1 710 | 8 |

(1) I.E = îlot-équivalent, correspondant à un îlot théorique de 150 µm de diamètre (nombres arrondis à la centaine).

(2)

$$\text{Index d'isolement} = \frac{\text{Nbre d'I.E}}{\text{Nbre d'îlots}}$$

(3)

$$\text{Rendement de purif.} = \frac{\text{Nbre d'I.E récoltés après purification}}{\text{Nbre d'I.E déposés sur le gradient}}$$

(4)

$$\text{Rendement final} = \frac{\text{Nbre d'I.E récoltés après purification}}{\text{Masse de pancréas digéré}}$$

(nombres arrondis à la dizaine)

(5)

$$\text{Taux de pureté} = \frac{\text{Nbre d'îlots}}{\text{Nbre total d'amas cellulaires}}$$

dans la suspension finale

Les rendements de digestion (5000 îlots-équivalents/g de pancréas) et le rendement global (3600 îlots-équivalents/g de pancréas) sont comparables aux meilleurs résultats publiés (respectivement 5580 et 3410 : Kerr-Conte J et al., Transplantation Proceedings 1994 ; 26 (2) 614-5). Le rendement de purification (85%) et l'index d'isolement (1,8) se situent au-dessus des performances publiées (45% et de l'ordre de 1, respectivement : Kerr-Conte J et al., Transplantation Proceedings 26 (2) : 614-5 ; 74% : Chadwick DR et al., Horm Metab. Res. 1994 ; 26 (1) : 64).

L'invention n'est pas limitée aux modes de réalisation représentés et elle est susceptible de variantes. En particulier, la forme des éléments constitutifs du dispositif décrit plus haut, cuve cylindrique pour le récipient 1, tambour pour le conteneur 5 ne doit pas être considérée comme limitative. En particulier, le conteneur pourrait aussi bien avoir la forme d'un prisme droit ou d'une sphère.

**Revendications**

1. Dispositif de digestion d'au moins un organe ou d'une partie d'organe en vue d'en extraire des cellules ou amas de cellules d'intérêt biologique comprenant:

   - une enceinte (1,2), et
   - des moyens d'agitation mécanique pour favoriser la désagrégation d'un organe soumis à l'action d'un milieu de digestion biologique,

   caractérisé en ce que les moyens d'agitation mécanique comportent des moyens (6) pour entraîner en rotation au moins partielle sur lui-même un organe placé dans l'enceinte (1,2).

2. Dispositif selon la revendication 1, caractérisé en ce que les moyens pour entraîner en rotation un organe placé dans l'enceinte (1,2) comprennent un moteur (6) pouvant être couplé à l'enceinte (1,2) pour faire tourner l'enceinte (1,2) autour d'un axe sensiblement horizontal.

3. Dispositif selon la revendication 1, caractérisé en ce que les moyens pour entraîner en rotation un organe placé dans l'enceinte comprennent :

- un conteneur (5) disposé dans l'enceinte (1,2) pour contenir l'organe, délimitant une première chambre située à l'extérieur du conteneur et une deuxième chambre à l'intérieur du conteneur (5);
- des moyens (6) pour faire tourner le conteneur (5) autour d'un axe, et
- des moyens de communication sélectifs (23) entre la première chambre et la deuxième chambre pour permettre le passage de liquide de la première chambre vers la deuxième chambre et le passage de fragments d'organe de la deuxième chambre vers la première chambre.

4. Dispositif selon la revendication 3, caractérisé en ce que le conteneur (5) a un axe de symétrie disposé sensiblement horizontalement.

5. Dispositif selon une des revendications 3 ou 4, caractérisé en ce que le conteneur (5) a sensiblement la forme d'un tambour ayant deux parois d'extrémité sensiblement circulaires (21,22) jointes par une paroi tubulaire (23).

6. Dispositif selon une des revendications 3 à 5, caractérisé en ce que les moyens pour faire tourner le conteneur comprennent un moteur (6) pouvant être couplé au conteneur (5) pour le faire tourner autour de son axe de symétrie.

7. Dispositif selon une des revendications 3 à 6, caractérisé en ce que les moyens de communication sélectifs comprennent au moins une portion d'une paroi (23) du conteneur (5) constituée par un treillis.

8. Dispositif selon les revendications 3 et 7, caractérisé en ce que la paroi tubulaire (23) du conteneur (5) est constituée par un treillis.

9. Dispositif selon une des revendications 2 à 8, caractérisé en ce que l'enceinte comprend un récipient (1) ayant une ouverture pour introduire l'organe à digérer située dans un plan sensiblement vertical.

10. Dispositif selon une des revendications 3 à 8, caractérisé en ce que l'enceinte comprend un récipient (1) ayant une ouverture pour introduire l'organe à digérer située dans un plan sensiblement horizontal.

11. Dispositif selon une des revendications 3 à 10, caractérisé en ce qu'il comprend des moyens pour brasser un liquide dans la première chambre.

12. Dispositif selon les revendications 3 et 11, caractérisé en ce que les moyens pour brasser le liquide dans la première chambre comprennent des aillettes montées sur une surface extérieure du conteneur et s'étendant dans la première chambre.

13. Dispositif selon une des revendications 1 à 12, caractérisé en ce qu'il comporte des moyens (26,29) pour régler la température du contenu de l'enceinte (1,2) comprenant:

- une chemise (26) entourant au moins partiellement l'enceinte (1,2),
- des moyens pour faire circuler un liquide dans la chemise (26), et
- des moyens (29) pour régler la température du liquide mis en circulation dans la chemise (26).

14. Procédé pour isoler des cellules ou des amas de cellules d'intérêt biologique à partir d'un organe ou d'une partie d'organe comprenant les étapes de :

- injecter dans l'organe un liquide de digestion apte à provoquer une désagrégation du tissu conjonctif enserrant les cellules d'intérêt biologique
- placer l'organe dans une enceinte (1,2) et remplir l'enceinte (1,2) de liquide
- agiter l'organe dans l'enceinte (1,2) pendant la digestion ;
- vidanger le contenu de l'enceinte (1,2) lorsque la digestion est achevée ;
- séparer les cellules d'intérêt biologique du contenu de l'enceinte (1,2) ;

caractérisé en ce que l'étape d'agitation de l'organe consiste à soumettre l'organe à un mouvement de rotation au moins partielle sur lui-même jusqu'à sa désagrégation.

15. Procédé selon la revendication 14, caractérisé en ce que l'organe est soumis à un mouvement de rotation continu, toujours dans le même sens.

16. Procédé selon la revendication 14, caractérisé en ce que l'organe est soumis à un mouvement de rotation alternatif, tantôt dans un sens tantôt dans l'autre sens.

17. Procédé selon une des revendications 14 à 16, caractérisé en ce que le liquide de digestion est porté à une température optimale de digestion préalablement à son introduction dans l'enceinte (1,2) et est maintenu à cette température pendant la digestion.

18. Procédé selon une des revendications 14 à 17, caractérisé en ce que, après l'étape de vidange, l'enceinte (1,2) est rincée puis vidangée à nouveau.

19. Procédé selon une des revendications 14 à 18, caractérisé en ce que l'enceinte (1,2) est vidangée par gravité.

20. Procédé selon l'une des revendications 14 à 19, caractérisé en ce que l'étape de séparation des cellules d'intérêt biologique comprend une étape préalable de filtration du contenu de l'enceinte (1,2).

21. Procédé selon une des revendications 14 à 20, caractérisé en ce que l'étape de séparation des cellules d'intérêt biologique comprend une étape de centrifugation pour séparer un culot de centrifugation contenant les cellules ou amas de cellules d'intérêt biologique, d'une phase liquide ne contenant sensiblement pas de cellules ou d'amas de cellules d'intérêt biologique.

22. Procédé selon la revendication 21, caractérisé en ce que l'étape de séparation des cellules d'intérêt biologique comprend en outre les étapes de

- suspendre le culot de centifugation dans un liquide de conservation ; et
- centrifuger la suspension obtenue sur un gradient de densité continu pour séparer les cellules ou amas de cellules d'intérêt biologique.

23. Procédé selon une des revendications 14 à 22, caractérisé en ce que le temps s'écoulant entre le début de l'étape d'injection de liquide de digestion dans l'organe et le début de l'étape de vidange du contenu de l'enceinte (1,2) a une durée fixe prédéterminée.

Fig. 1

Fig. 2

Fig. 3

**Office européen des brevets**

# RAPPORT DE RECHERCHE EUROPEENNE

Numero de la demande

EP 96 42 0009

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | FR-A-2 696 754 (HOSPAL INDUSTRIE ET I.N.S.E.R.M.)<br>* revendications; figures *<br>--- | 1-23 | C12M3/08 |
| Y | US-A-5 254 472 (E.H. BROOKS & J.A. BROOKS.)<br>* revendications; figures *<br>--- | 1-23 | |
| Y | GB-A-1 356 794 (THE SECRETARY OF STATE FOR DEFENCE) 12 Juin 1974<br>* page 2, colonne 2, ligne 113 - page 2, colonne 1, ligne 48; revendications; figures *<br>--- | 1-13 | |
| Y | US-A-4 264 741 (M.R. FRIEDMAN & T.E. STINETT)<br>* colonne 2, ligne 38 - colonne 3, ligne 44; revendications; figures *<br>--- | 1-13 | |
| A | WO-A-95 01419 (BAXTER INTERNATIONAL INC.)<br>--- | | |
| A | EP-A-0 236 049 (CONNAUGHT LABORATORIES LIMITED)<br>----- | | **DOMAINES TECHNIQUES RECHERCHES (Int.Cl.6)**<br><br>C12M |

Le présent rapport a été établi pour toutes les revendications

| Lieu de la recherche | Date d'achèvement de la recherche | Examinateur |
|---|---|---|
| LA HAYE | 24 Avril 1996 | Bevan, S |

EPO FORM 1503 03.82 (P04C02)